# EUROPEAN PATENT SPECIFICATION

(11) **EP 2 341 954 B1**
(45) Date of publication and mention of the grant of the patent: **27.05.2015**
(21) Application number: 09785593.6
(22) Date of filing: 08.09.2009
(51) Int. Cl.: A61L 29/14, A61L 31/10, A61L 31/14, A61L 29/08

(54) **POLYMERIC MATERIAL**
POLYMERMATERIAL
MATÉRIAU POLYMÈRE

(30) Priority: 08.09.2008 GB 0816365
(43) Date of publication of application: 13.07.2011
(73) Proprietor: Laboratorios Farmacéuticos Rovi, S.A., 28037 Madrid (ES)
(72) Inventor: ANDREWS, Gavin P, Belfast Antrim BT9 7BL (GB); JONES, David S, Belfast Antrim BT9 7BL (GB); GORMAN, Sean P, Belfast Antrim BT9 7BL (GB)
(74) Representative: Elzaburu Marquez, Alberto
(86) International application number: PCT/GB2009/051134
(87) International publication number: WO 2010/026433

(56) References cited:
- WO-A1-89/05671
- WO-A2-2006/013374
- US-A1- 2004 062 778
- US-B1- 6 306 422

## Description

### Field on invention

The invention relates primarily to the field of medical devices. More specifically, the invention pertains to medical devices comprising pH sensitive degradable layers, methods of making medical devices containing pH sensitive layers and methods of using medical devices containing pH sensitive degradable layers.

### Background

The use of medical devices inserted into a patient's body is now routine in healthcare management within hospitals and nursing homes. Although there are substantial benefits associated with the use of inserted medical devices, such as, for example, catheters and stents, there are very worryingly a number of potentially dangerous complications that may lead to an increase in the time patients remain in hospital and more importantly in an increase in the number of patient deaths associated with the use of these devices. These complications arise principally because of the way in which a patient's body reacts to insertion of a medical device and what it perceives to be a foreign object. Consequently patients are often plagued by infection associated with the insertion of a medical device and this is seen to be one of the most critical disadvantages of an otherwise highly effective and beneficial medical treatment. There is an urgent need to improve what is often referred to as device-related infection.

Typically device-related infection begins with bacterial adherence, developing with the formation of biofilm. Bacteria and pathogens which typically colonise catheters produce urease which degrades urea in urine to form carbon dioxide and ammonia. At increased pH associated with such degradation, minerals in urine precipitate leading to encrustation.

Catheter encrustation can cause blockage of the catheter leading to an increase in the frequency with which the catheter must be removed and replaced. Encrustation also results in an increase in the pain of removal of the catheter. The tissue surrounding the catheter is also far more likely to become infected. This is particularly problematic for patients requiring long term catheterisation. Serious consequences include septicaemia, pyelonephitis and shock.

Additionally, associated pathogens within the biomass can compromise medical device lifetime through the expression of potent urease isoenzymes, which act to alkalinise urine through the conversion of urea to ammonia and carbon dioxide.

Previous approaches to overcoming this problem include the incorporation of antibiotics into the device to combat infection. After insertion, therapeutic agents may be released by diffusion and ultimately reside locally in the biological fluid adjacent to the device thus preventing bacterial adherence. Further, attempts have been made to modify device surfaces to reduce their susceptibility to infection.

Despite the attempts to alleviate the complications that plague the use of urinary devices, many problems still exist. Therefore, whilst antibiotic therapies and novel surface coatings may provide a temporary resolution, the only real definitive solution to the problems associated with urinary catheterisation and ureteral stenting is device removal.

Medical devices, such as catheters, coated in lubricants are known. Lubricants comprising cross-linked hydrogels, including carboxylic acid functional groups are also known.

US 6306422 discloses a device, particularly a urinary catheter, coated in a cross linked polymer hydrogel. At a trigger pH, the polymer swells through absorption of water. This absorption of water increases pore size of the hydrogel, enhancing the release of an active in a sustained release fashion. The polymer hydrogel of US 6306422 remains water insoluble throughout, and remains coated onto the device. The active is typically one or more of an antibiotic and a urease inhibitor. The release of these actives may control bacterial surface growth and control the formation of encrustation respectively. However, the release of a urease inhibitor does not remove any encrustation which has already formed on the catheter. In addition, the actives released from devices disclosed in US 6306422 would not be able to penetrate the biofilm formed by bacteria to remove existing bacterial colonisation.

US2004/0062778 A1 discloses compositions and devices coated with a pH sensitive material to release an active ingredient when the pH of the surrounding environment reaches a desired level.

WO 2006/013374 A2 discloses medical devices capable of releasing a medically active ingredient comprising a pH sensitive polymer for releasing said ingredient in the presence of a solution having a pH within a predetermined range.

### Summary of invention

The inventors have developed a device surface that is inherently resistant to infection through the use of intelligent in-vivo reactions and preferably impregnation with antibiotics. In particular, the device surface comprises shedding biomaterials, which can be shed in response to pH changes, as an alternative to currently utilised materials.

According to the present invention there is provided a device comprising a body structure having one or more surfaces comprising at least one pH sensitive degradable layer wherein the at least one pH sensitive degradable layer comprises a pH sensitive polymer wherein the pH sensitive degradable layer is capable of controlled degradation at a defined pH. The pH sensitive layer incorporates buffer groups to reduce the rate of dissolution or erosion.

According to the present invention, there is provided a device comprising a body structure having one or more surfaces wherein at least one of the surfaces comprises a pH sensitive layer comprising a linear polymer, wherein the water solubility of the linear polymer increases from a first water solubility to a second water solubility at a pH trigger.

Suitably, in embodiments, the device can be any device wherein the pH of fluid, for example bodily fluid, surrounding the device increases or decreases from a defined value, for example wherein pH can increases or decreases from physiological pH in response to infection.

At the pH trigger, the linear polymer ionises, and this causes the water solubility of the polymer to increase. The ionised linear polymer then dissolves into the aqueous environment surrounding the device, and a new surface of the device is revealed. The new surface does not have any bacteria colonised thereon, and will be free of encrustation. The device of the present invention can thus remain implanted for extended periods of time compared to prior art devices. The risk of infection and encrustation is also reduced as the surface of the device of the present invention is shed once colonised with bacteria to any significant extent, and a new surface is exposed.

The device of the present invention may typically be a urinary catheter or urinary stent. Bacteria which typically colonises such devices release urease. Urine degrades into ammonia and carbon dioxide upon contact with urease, substantially increasing the pH of the area surrounding the catheter. This increase in pH causes minerals to precipitate from urine, causing encrustation of the catheter. The increase in pH generated through the production of ammonia triggers the linear polymer of the present invention to ionise, and the water solubility of the polymer increases accordingly. The linear polymer dissolves into the surrounding aqueous environment, and any encrustation present on the outer surface of the device is removed with the linear polymer. A new surface of the device is exposed. The new surface is free of bacteria and encrustation.

Alternatively, the device of the present invention may be in the form of dental braces or dentures. Upon bacterial colorisation of such devices, the surrounding pH decreases to below physiological pH. As bacteria in the oral cavity produce acid, the greater the degree of bacterial colorisation, the greater the amount of acid produced, lowering the surrounding pH.

The linear polymer of the present invention can be capable of changing from providing a stable layer to a layer which undergoes controlled erosion or dissolution as the pH of the surrounding environment moves away from physiological pH, wherein physiological pH is typically 6.2. Suitably, such erosion or dissolution occurs towards the endpoints of the range pH 5.5 to pH 7. In embodiments, a pH sensitive polymer can be capable of controlled degradation or erosion at a pH indicative of infection, being removed from physiological pH, for example pH 7.0.

The pH trigger depends on the intended environment surrounding the device of the present invention. Where the device is intended to be implanted into a neutral or alkali environment such as the urinary bladder, the pH trigger is typically above 6.5; suitably above 7; more suitably approximately 7.2. Where the device is intended to be implanted into an acidic environment such as the stomach, the pH trigger is typically less than 6.0; suitably less than 5.5.

Where the pH of the area surrounding the device of the present invention moves away from the trigger pH and towards physiological pH, the water solubility of the pH sensitive layer may decrease accordingly, and move towards the first water solubility. As such, the rate of dissolution or erosion of the pH sensitive layer may decrease. According to one embodiment, the pH sensitive layer dissolves or erodes only where the device is colonised by bacteria. The dissolution or erosion of the pH sensitive layer may start and stop depending on the colonisation of the device.

Suitably, in embodiments, such a pH sensitive layer can provide for a first rate of release of functional excipients at physiological pH (for example pH 6.2) and at a second rate at non physiological pH (for example pH 7.0). Generally the first rate is lower than the second rate. In embodiments where shedding of the pH sensitive layer is minimal at physiological pH and increased at a pH removed from physiological pH, elution of the functional excipients may be correlated with erosion or dissolution of the pH sensitive layer. This can be advantageous as infection can move pH away from physiological values and thus the release of the functional excipients may correlate with infection.

Suitably, in preferred embodiments the linear polymer is biocompatible. A "biocompatible" material is a material that is compatible with living tissue or a living system by not being toxic or injurious. In particular embodiments the device may comprise material which forms biostable layers. A "non-bioabsorbable" or "biostable" material refers to a material, such as a polymer or copolymer, which remains in the body without substantial bioabsorption. Such layers may be included in the device of the present invention to provide structural support to the device.

Suitably, in embodiments, the linear polymer undergoes structural changes with respect to changes in pH, in particular the linear polymer may become ionised with changes in pH, causing the linear polymer to have increased water solubility.

The polymers for use in the device of the present invention are linear rather than cross-linked. The linear polymer of the present invention become ionised at a pH trigger, causing the water solubility of the linear polymer to greatly increase. In contrast, cross-linked polymers ionise at a pH trigger causing the polymers to absorb water and swell. The water solubility of the cross-linked polymer is not altered through ionisation, and even following ionisation the cross-linked polymer is retained on devices such as those disclosed in US 6306422. Such cross-linked polymers are generally in the form of hydrogels. The linear polymers of the device of the present invention are generally extrudable.

Typically, the pH sensitive layer absorbs less than 30 wt % water prior to ionisation; generally less than 20 wt%, suitably less than 10 wt%. In contrast, prior art cross-linked hydrogels for use in connection with devices such as those disclosed in US 6306422 absorb up to several thousand times their weight in water prior to ionisation. The water solubility of the hydrogels is not affected, and these hydrogels maintain their shape and do not dissolve into the surrounding aqueous environment. Although the polymers for use in the device of the present invention may swell with absorption of water prior to ionisation, this precedes ionisation resulting in an increase in water solubility and dissolution into the surrounding aqueous environment.

Where structural changes in the linear polymer are intended to be triggered at a pH of at least 6.5, the linear polymer typically comprises SO bonds and/or one or more of carboxylic groups and sulphate groups. Typically the linear polymer may be pH sensitive cellulose polymers, for example celllulose esters or cellulose ethers. In one embodiment, the linear polymer may be a methacrylate polymer or a polymer comprising methacrylate. According to one embodiment, the polymer has the following structure:

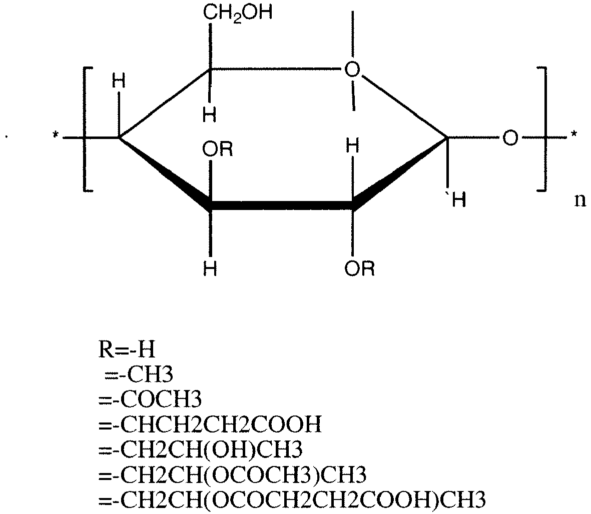

In embodiments of the invention the linear polymer can be selected from methacrylate polymers and cellulose polymers. In embodiments, the linear polymer can be selected from the group comprising, for example, Eudragit® L100, S100, HPMC-AS, HTMAC-P, and combinations of these polymers can be used. Polymers which could be suitably used to form the pH sensitive layer could be obtained from, for example Shin-Etsu, for example Shin-Etsu AQOAT, Degussa or the like.

In embodiments, the linear polymer can be a Eudragit® polymer. Eudragit® polymers can be provided as a single system or blends of two different types. In embodiments Eudragit® L100, S100 and combinations of these polymers can be used.

In embodiments, Eudragit® L100, can be used to provide a layer which is capable of erosion at pH values greater than 6.

In embodiments, Eudragit® S100 can be used to provide a layer which erodes at pH values exceeding 7.0.

As will be appreciated, pH sensitive layers of a device can be manufactured using a combination of both L100 and S100 to generate systems that erode slowly under normal urinary conditions, but will rapidly shed at higher pH values. Layers of different pH sensitive polymers for example layers of Eudragit® L100, S100 and combinations of these polymers can be used to form a device such that different layers in a device erode at different pH levels.

Where structural changes in the linear polymer are intended to be triggered at a pH of less than 6.0, the linear polymer typically comprises primary, secondary and tertiary amines, typically NH₂ groups; suitably the polymer comprises diethylaminoacrylate, dimethylaminoethylacrylate and/or other acrylate monomers. Typically the polymer is a copolymer of dimethylaminomethacrylate and other acrylate monomers. Suitably the polymer is that sold under the trade mark Eudragit® E100.

As noted above, the water solubility of the linear polymer of the device of the present invention increases from a first water solubility to a second water solubility at a pH trigger.

Typically at a pH of the pKa of the polymer, 50% of the polymer or more is ionised.

According to one aspect of the present invention the second water solubility of the linear polymer is at least 200% more than the first water solubility of the linear polymer, generally at least 400% more, typically at least 600% more.

Advantageously, upon dissolution or erosion the polymer chain of the linear polymer remains intact, comprising the same monomer units as before dissolution or erosion.

The device of the present invention may be any device wherein a change in pH is associated with colonisation of the device with bacteria. In embodiments of the present invention, the device is a medical device, for example an intracorporeal or extracorporeal device including catheters, temporary or permanent implants, stents, grafts, repair devices, and implantable devices.

Typically the device is a catheter, suitably a urinary catheter, a urethral stent, a naso-gastric tube, a CAPD tube, a bilary stent, dental braces or dentures.

Advantageously, the device of the present invention is a urinary catheter or a urethral stent.

The pH sensitive layer may comprise functional excipients to be released with the dissolution or erosion of the pH sensitive layer. The functional excipients may suitably be buffer groups such as citric acid, tartaric acid, succinic acid, and fumaric acid, antimicrobial compounds, Levofloxacin and Nalidixic acid, antibiotic compounds, chlorhexidine, povidone-iodine, tridosan, urease inhibitors, EDTA and plasticizing agents, for example triethyl citrate, and tributyl citrate or other standard excipients used to facilitate manufacture or performance.

Typically the functional excipients are released in a sustained release manner following implantation of the device. According to one embodiment, the rate of release of the functional excipients may increase sharply upon erosion or dissolution of the pH sensitive layer.

The functional excipient may be adsorbed directly to the linear polymer, or may be disposed inside the device or otherwise associated with it via the use of one or more linker molecules or other attachment means including covalent, ionic, van der Waals bonds. The pH sensitive layer and/or surface may be configured such that controlled release of the functional excipient occurs, for example the functional excipient elutes slowly over time. By "controlled release" is meant an alteration of the rate of release of a therapeutic agent or functional excepient from a medical device coating in a given environment. This may be accomplished using time released coatings, for example. In embodiments of the device of the invention, layers are provided which are adapted to simultaneously release therapeutic agent(s) at two or more different rates from different portions of a layer or at two different rates depending on the pH surrounding the device.

In embodiments, the device can include layers, which can include pH sensitive polymer layers, which are loaded with a functional excipient, in particular a drug, for example an antibiotic. Alternatively, in embodiments the medical device or the medical device coating comprising the pH sensitive layer degrades in a controlled manner relative to pH and the drug can be bound to the linear polymer. By incorporating a drug within the material of the medical device or the material coating the medical device, the drug is dispensed in a gradual manner as the layer comprising the pH sensitive polymer degrades.

Suitably, in embodiments the dissolution of a pH sensitive layer triggers the release of an antibiotic.

In embodiments, the device of the present invention can comprise a drug which minimises bacterial adhesion to the device or growth of a pathogen on the device, for example an antibiotic. An advantage of the device of the present invention is that it allows much higher drug concentrations at the site of infection in comparison to conventional routes of drug therapy such as orally swallowed tablets.

The release of an antibiotic may control bacterial growth on the surface of the device. However, a biofilm is generally formed once bacteria have effected colonisation of a device. Antibiotic compounds cannot generally penetrate such biofilms, and are therefore not very effective at removing such bacterial biofilms. The release of a urease inhibitor acts to control the growth of encrustation but does not remove encrustation which has already formed. The surface of the device of the present invention starts to dissolve or erode at a trigger pH, and bacterial colonisation and encrustation is removed with the surface. A new surface is revealed free of all bacterial colonisation and encrustation.

In effect a device of the present invention can self-cleanse once infected with bacteria, that is to say should drug elution fail to inhibit bacterial adherence and subsequently result in increased urinary pH by the action of urease on urea, a layer of the device is capable of recognising the formation of microbial biofilm and initiate controlled erosion (regulated by the incorporation of organic acids, such as citric acid) and thus remove any adherent masses. In so doing, the device surface will be cleansed and the functional agents (EDTA & citric acid) incorporated into the film will be released to the device/fluid interface. This will regulate the urinary pH by the action of citric acid and very importantly sequester Ca²⁺ and Mg²⁺ metal ions. This process will renew the device surface, return the pH to normal values and 'mop up' metal ions that are pertinent to the formation of crystalline deposits on the device surface.

This enables the devices and ultimately the patients to remain free from infection for the duration the device is to be used.

Upon release of the functional excipients, the bacterial colonisation of the device may be reduced, and the pH of the area surrounding the device may move away from the trigger pH and towards physiological pH. The water solubility of the pH sensitive layer may decrease towards the first water solubility accordingly.

The linear polymer absorbs water at a trigger pH, causing ionisation. The rate of ionisation may be engineered by controlling the rate of absorption of water, typically by controlling the density of the pH sensitive polymer layer. A decreased density of linear polymer in the pH sensitive layer leads to a decreased rate of ionisation.

The rate of ionisation depends on the composition of the pH sensitive layer, as well as the pH of the area surrounding the device.

According to one embodiment the pH sensitive layer comprises a second or third hydrophilic polymer. Suitable hydrophilic polymers include polyethylene oxide, polyacrylic acids and/or cellulose derivatives (particularly linear cellulose derivatives) such as hydroxypropylcellulose, hydroxypropylmethylcellulose and polyvinylpyrrolidone. The addition of one or more hydrophilic polymers to the pH sensitive layer provides physical interactions such as Van der Waals interactions.

Alternatively, the pH sensitive layer may comprise a hydrophobic polymer, in particular a low molecular weight hydrophobic polymer. Suitable hydrophobic polymers include polylactic acid, polyglycolic acid, polylactide-co-glycolide and polycaprolactone. Generally the hydrophobic polymer is substantially homogenously dispersed in the pH sensitive layer. The rate of dissolution or erosion of the pH sensitive layer is dependent on its composition. Buffer groups are incorporated into the pH sensitive layer to reduce the rate of dissolution or erosion. Suitable buffer groups include citric acid, tartaric acid, succinic acid, fumaric acid and related compounds. Where the extent of bacterial colonisation is low, the number of ions released is low. Some of these ions will be taken up by the buffer group resulting in a lower rate of degradation, and increasing the lifetime of the device of the present invention. The number of ions released will increase with increased bacterial colonisation leading to the erosion or dissolution of the pH sensitive layer regardless of the incorporation of the buffer group.

According to one aspect, the device of the present invention may comprise more than one pH sensitive layer; typically more than three pH sensitive layers; more suitably five pH sensitive layers.

Each pH sensitive layer may have the same pH trigger or a different pH trigger.

Each pH sensitive layer may have a different second water solubility. Alternatively each pH sensitive layer may have the same second water solubility.

Each pH sensitive layer may have the same rate of ionisation and the same rate of dissolution or erosion. Alternatively, different pH sensitive layers may have the same or different rates of ionisation and/or rates of dissolution or erosion.

According to one embodiment, adjacent pH sensitive layers may have the same pH trigger, but different second water solubilities.

Alternatively, adjacent pH sensitive layers may have different pH triggers, but the same second water solubilities.

Typically adjacent pH layers have different rates of ionisation, or different rates of dissolution or erosion.

According to one embodiment, different pH sensitive layers comprise different functional excipients to be released upon dissolution or erosion of the pH sensitive layer.

According to one embodiment, the device may comprise a lubricating layer to increase its ease of insertion and removal. Typically the lubricating layer may comprise one or more cross-linked polymers.

Generally the device comprises an inside surface and an outside surface, said inside surface defining a lumen.

Typically the outside surface of the device comprises the lubricating layer. Generally the inside surface of the device comprises the pH sensitive layer.

Generally the device comprises at least one structural layer which is substantially non-degradable or erodable in the body and provides structural stability to the device regardless of the pH of the surrounding environment.

Typically the water solubility of the structural layer remains substantially constant between a pH of 2 to 10. Generally the water solubility of the structural layer remains substantially constant regardless of the pH of the surrounding environment.

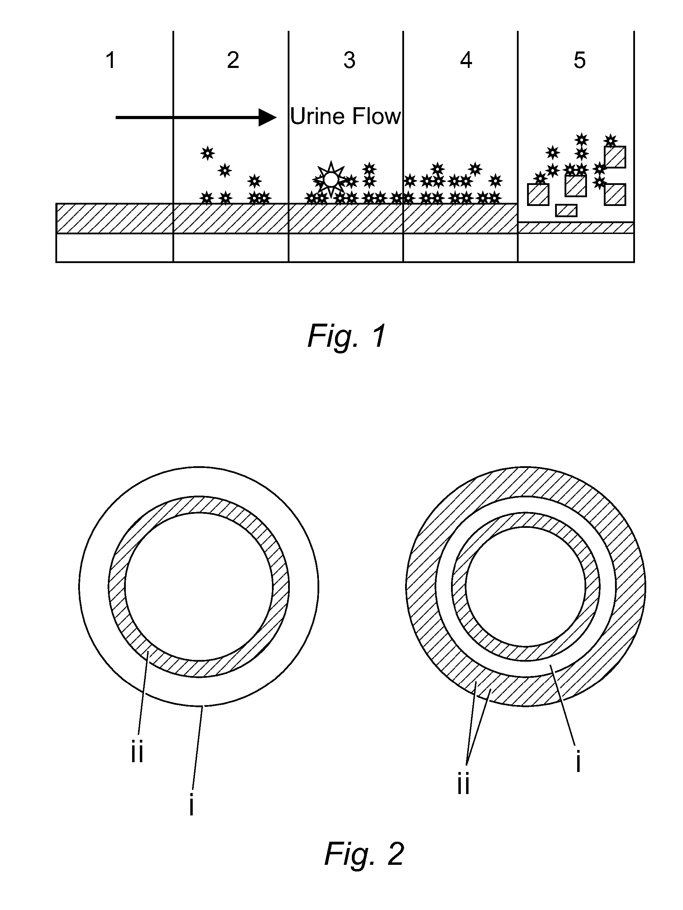

In particular embodiments the device can comprise a two layer system wherein the pH sensitive layer, for example a Eudragit® layer is provided on the inside of a two layer system. In such a system, a fluid, for example a bodily fluid such as urine, can flow through the inner lumen of the device (Figure 2a).

In alternative embodiments the device can comprise a three layer system in which two pH sensitive layers, for example Eudragit® layers, form the inner and outer layers of the device. In such three layer systems, a fluid, for example a bodily fluid such as urine, can flow through the inner lumen and over the outer surface of the device (Figure 2b).

In further alternative embodiments, devices comprising a plurality of pH sensitive layers can be provided. In particular embodiments a first pH sensitive layer can be provided adjacent to a second pH sensitive layer such that on erosion of the first layer, the second layer is exposed. In embodiments, the pH sensitive layers can be capable of erosion at different pH values.

Suitably, in embodiments the inner and outer layers of the device can be melt extruded.

Suitably, in embodiments a degradable layer can be amenable to insertion and removal of the device from within the body of a patient. In embodiments, where necessary, a structural layer comprising suitable polymers can be provided in combination with a degradable layer in a device.

According to a further aspect of the present invention there is provided a coating for application to a device, the device comprising a body structure having one or more surfaces and the coating being adapted to be applied to at least one surface of the device such that when a surface of the device is provided with at least one coating, a pH sensitive layer is provided on the device, said pH sensitive layer comprising a linear polymer wherein the water solubility of the linear polymer increases from a first water solubility to a second water solubility at a pH trigger.

In particular embodiments the device can comprise a coating comprising a plurality of pH sensitive layers.

The term "coating," as used herein and unless otherwise indicated, refers generally to material attached to a device. A coating can include material covering any portion of a medical device, and can be configured as one or more coating layers. A coating can have a substantially constant or a varied thickness and composition. Coatings can be adhered to any portion of a device surface, for example a medical device including the luminal surface, the abluminal surface, or any portions or combinations thereof.

Generally by pH sensitive layer is meant a layer which can be dissolved or eroded at a defined pH, for example wherein the polymer can be ionised at higher pH levels such that the water solubility of the polymer increases. Suitably, after sufficient dissolution or erosion, a complete layer may be removed such that a new layer in the device is exposed. Generally upon dissolution or erosion of the pH sensitive layer, the polymer chain remains intact with the same monomer units.

In embodiments, the pH sensitive layer includes functional excipients such as citric acid or other small organic molecules and such functional excipients will be released upon dissolution or erosion of the pH sensitive layer, generally in a controlled release fashion.

A method of forming a device could comprise the steps of providing a structural layer and applying at least one pH sensitive layer thereto, said pH sensitive layer comprising a linear polymer wherein the water solubility of the linear polymer increases from a first water solubility to a second water solubility at a pH trigger.

Typically the structural layer has an inside surface and an outside surface, said inside surface defining a lumen. Generally the pH sensitive layer is applied to the inside surface of the structural layer. The method may comprise the step of applying more than one pH sensitive layer.

The device is a medical device, suitable for insertion or implantation into the human or animal body.

In particular embodiments of the second aspect of the invention, the method includes multi-layer extrusion.

A method of preventing or mitigating infection associated with a device implanted or inserted into the human or animal body could comprise the step of implanting or inserting a device into the human or animal body, said device comprising a pH sensitive layer comprising a linear polymer, wherein the water solubility of the linear polymer increases from a first water solubility to a second water solubility at a pH trigger.

Generally the method results in any infection already formed being removed.

Generally the method includes the step of preventing or mitigating the formation of encrustation of the device. Typically the method also includes the step of the removal of any encrustation already formed.

Typically the time for which the device is implanted or inserted into the human or animal body without associated infection is at least 1 day, generally at least 3 days, suitably 7 days or more.

The time of implantation or insertion may be increased by at least 100% compared to equivalent devices which do not comprise at least one pH sensitive layer. Generally the time of insertion or implantation may be increased by at least 150%; typically at least 200%.

According to one embodiment, the device implanted is as described above.

Typically the method of the present invention prevents or mitigates infection associated with the insertion or implantation of a catheter, in particular a urinary catheter, a stent, in particular a urethral stent or a bilary stent, an implantable or insertable tube, in particular a naso-gastric tube or a CAPD tube, dental braces or dentures.

A method of preventing or mitigating infection associated with a device implanted or inserted into the human or animal body could comprise the steps of applying at least one pH sensitive layer to the device, said pH sensitive layer comprising a linear polymer wherein the water solubility of the linear polymer increases from a first water solubility to a second water solubility at a pH trigger.

Typically the device comprising the pH sensitive layer is as described above.

A device for use in therapy comprises at least one pH sensitive layer comprising a linear polymer wherein the water solubility of the linear polymer increases from a first water solubility to a second water solubility at a pH trigger.

Typically the device is as described above.

Generally the therapy is preventing or mitigating infection associated with the insertion or implantation of the device in a human or animal body.

A pH sensitive polymer shows a variable drug elution profile in the pH range pH 5 to pH 7.8, more preferably in the pH range pH 6 to pH 7.2 or alternatively in the pH range 5 to 6, preferably 5.5 to 6. The pH sensitive polymer is linear.

By a variable drug elution profile is meant that drug is eluted from a polymer at a first rate at a first end of a given pH range and a second different rate at a second opposite end of a given pH range. As a pH sensitive polymer undergoes degradation, for example becomes more soluble at a given pH, for example a pH removed from physiological pH, drug release can occur. The greater the degradation, for example at more extreme pH values removed from physiological pH, the greater the release of drug.

A pH sensitive polymer has a change in structural integrity in the pH range pH 5 to pH 7.8, more preferably in the pH range pH 6 to pH 7.2 or alternatively in the pH range 5 to 6, preferably 5.5 to 6. The pH sensitive polymer is generally linear.

By change in structural integrity it is meant the polymer is able to form sheets or layers of polymer about one end of the pH range, but is degraded and unable to form sheets or layers of polymer at an opposite end of the pH range.

Methods of use for treating patients with any one or more of the medical devices disclosed herein include, for example, a method of therapeutically treating a patient comprising contacting the patient with a medical device comprising a body structure having one or more surfaces comprising at least one pH sensitive layer wherein the at least one pH sensitive layer comprises a linear polymer wherein the water solubility of the linear polymer increases from a first water solubility to a second water solubility at a pH trigger. Methods for administering a drug compound to a body of a patient comprises, for example, providing a drug-eluting device of the present invention.

A method of administering a composition to a patient comprises providing a composition-eluting device, and introducing the composition-eluting device into the body of the patient, wherein the composition-eluting device comprises a body structure having one or more surfaces comprising at least one pH sensitive layer wherein the at least one pH sensitive layer comprises a linear polymer wherein the water solubility of the linear polymer increases from a first water solubility to a second water solubility at a pH trigger.

Throughout the specification, unless the context demands otherwise, the terms 'comprise' or 'include', or variations such as 'comprises' or 'comprising', 'includes' or 'including' will be understood to imply the includes of a stated integer or group of integers, but not the exclusion of any other integer or group of integers.

Preferred features and embodiments of each aspect of the invention are as for each of the other aspects mutatis mutandis unless context demands otherwise.

Embodiments of the present invention will now be described, by way of example only, with reference to the accompanying figures in which:
Figure 1 illustrates the steps of bacterial colonisation of a catheter of the present invention wherein colonising bacteria illustrated by * begin to colonise the device following insertion (1), such that the surface becomes colonised (2), and a microbial biofilm is formed (3), the urinary pH is increased by Urea-splitting bacteria (4), and erosion of Eudragit® occurs at elevated pH leading to removal of biofilm and insoluble deposits (5);
Figure 2 illustrates the drug eluting/self-cleansing layer (i) and the functional layer imparting structural integrity to the device (ii) of a two layer system (a) and a three layer system (b);
Figure 3 illustrates torque on the screw for a polymer that dissolves at pH 7 with a 5, 10 and 20% loading of the quinolone antibiotic Nalidixic Acid;
Figure 4 illustrates mechanical properties of formulations can be examined using DMTA : or Dynamic Mechanical Thermal Analysis in tension mode;
Figure 5 illustrates the release profile of 10% Nalidixic Acid from a device having a pH sensitive layer at pH 6 and pH 7, pH 6 taken to represent healthy uninfected urine; and

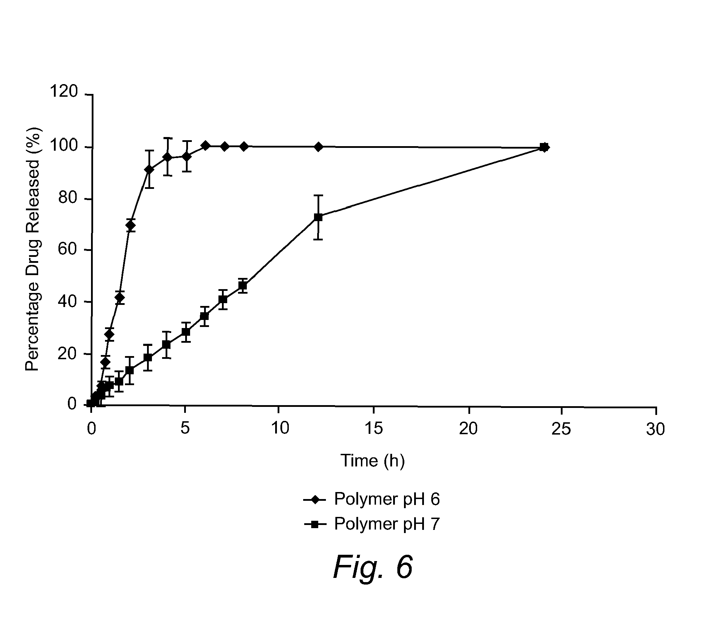

Figure 6 illustrates the release profile of 10% Nalidixic Acid from a device having a pH sensitive layer at pH 6 and pH 7.
Figure 7 illustrates the release profile of antimicrobial Levofloxacin from a device having a pH sensitive layer of Eudragit® L100 comprising 5% Levofloxacin at pH 6.2 and pH 7.8;
Figure 8 illustrates the release profile of antimicrobial Levofloxacin from a device having a pH sensitive layer of Eudragit® L100 comprising 10% Levofloxacin at pH 6.2 and pH 7.8;
Figure 9 illustrates the release profile of antimicrobial Levofloxacin from three devices having a pH sensitive layer of Eudragit® L100 comprising 5%, 10% and 20% Levofloxacin respectively;
Figure 10 illustrates the release profile of antimicrobial Levofloxacin from three devices having a pH sensitive layer of Eudragit® 4155F comprising 5%, 10% and 20% Levofloxacin respectively;
Figure 11 illustrates the release profile of antimicrobial Levofloxacin from a device having a pH sensitive layer of Eudragit® 4155F comprising 5% Levofloxacin at pH 6.2 and pH 7.8;
Figure 12 illustrates the release profile of antimicrobial Levofloxacin from a device having a pH sensitive layer of Eudragit® 4155F comprising 10% Levofloxacin at pH 6.2 and pH 7.8;
Figure 13 illustrates the release profile of antimicrobial Levofloxacin from a device having a pH sensitive layer of Eudragit® 4155F comprising 20% Levofloxacin at pH 6.2 and pH 7.8;
Figure 14 illustrates the release profile of antimicrobial Levofloxacin from a first device having a pH sensitive layer of Eudragit® 4155F and a second device having pH sensitive layer of Eudragit® L100 at pH 6.2 for 2 hours, pH 7.8 for 2 hours and pH 6.2 for 2 hours.
Figure 15 illustrates the release profile of antimicrobial Levofloxacin from a first device having a pH sensitive layer of Eudragit® 4155F and a second device having pH sensitive layer of Eudragit® L100 at a pH of 7.8 for 2 hours, pH 6.2 for 2 hours and pH 7.8 for 2 hours;
Figure 16 illustrates mean percentage mass over time at pH 6.2 for a first device having a pH sensitive layer of Eudragit® 4155F comprising 10% CA and a second device having a pH sensitive layer of Eudragit® 4155F comprising 10% CA and 10% Nalidixic acid;

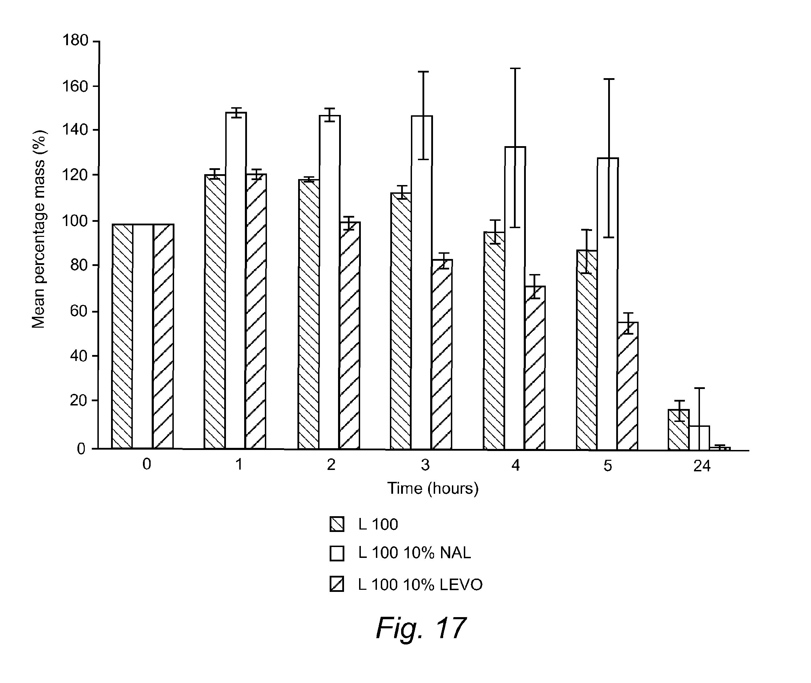

Figure 17 illustrates mean percentage mass over time at pH 6.2 for a first device having a pH sensitive layer of Eudragit® L100, a second device having a pH sensitive layer of Eudragit® L100 comprising 10% Nalidixic acid and a third device having a pH sensitive layer of Eudragit® L100 comprising 10% Levofloxacin;

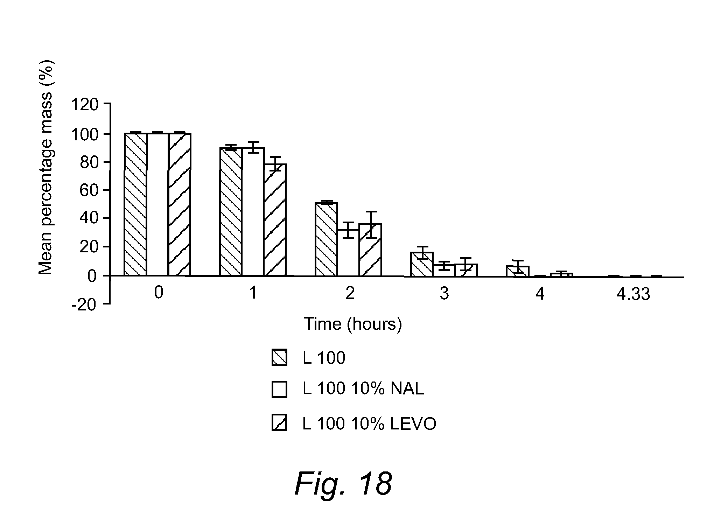

Figure 18 illustrates mean percentage mass over time at pH 6.2 for a first device having a pH sensitive layer of Eudragit® L100, a second device having a pH sensitive layer of Eudragit® L100 comprising 10% Nalidixic acid and a third device having a pH sensitive layer of Eudragit® L100 comprising 10% Levofloxacin;

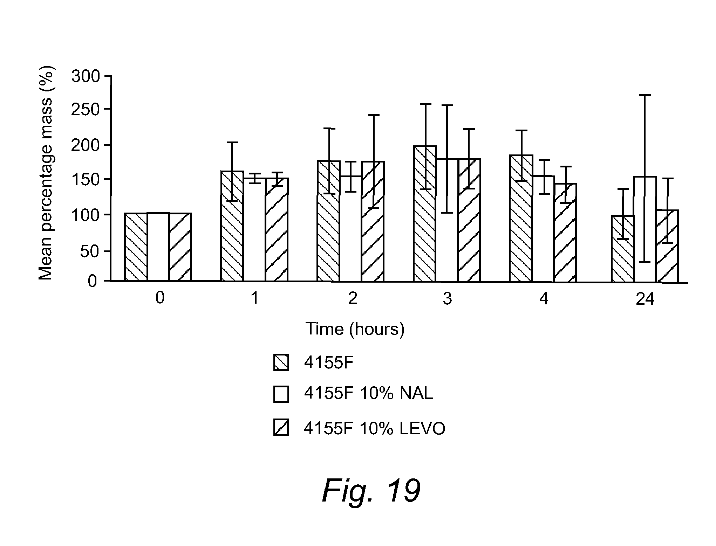

Figure 19 illustrates mean percentage mass over time at pH 6.2 for a first device having a pH sensitive layer of Eudragit® 4155F, a second device having a pH sensitive layer of Eudragit® 4155F comprising 10% Nalidixic acid and a third device having a pH sensitive layer of Eudragit® 4155F comprising 10% Levofloxacin;
Figure 20 illustrates the increased bacterial adherence of PMIR to a first device formed from PVC after 4 hours immersion in artificial urine compared to a second device having a pH sensitive layer of Eudragit® 4155F after 4 hours immersion in artificial urine.

### Detailed Description

Polymers were mixed with suitable plasticizers to enable processing with a twin-screw extruder. Different drug loadings of different antibacterial agents were then mixed with the polymer/plasticizer formulations. Formulations were stored in a dessicator for 24 hours prior to processing. The formulations were then extruded with varying concentrations of antibacterial agents. The samples were suspended in release medium appropriate to the in-vivo conditions. Samples were then filtered using 0.45 µm syringe filters and analysed using UV spectroscopy to determine their drug release properties.

It is expected that single drug loaded Eudragit® films will be manufactured using a twin-screw extrusion system that possess the ability to feed the antimicrobial, EDTA and citric acid at four different ports along the extruder barrel. This coupled with the modular design of the screw will allow products with extremely uniform density and homogeneity to be produced without degradation of the functional excipients.

Once manufactured the films will be subsequently characterisation and selection of optimised film layers for co-extrusion with PVC will be undertaken. Multi-layer extrusion of PVC and the optimised pH responsive layers will be performed on state-of-the-art multi-layer sheet extrusion facilities. Whilst typical urinary devices tend to be tubular, multi-layered sheets will be extruded to allow for testing.

Prior to co-extrusion, drug loaded Eudragit® pellets will be prepared using an air-cooled die face pelletiser connected to a twin-screw kneader. These pellets will be used to investigate the effects of plasticizer type, plasticizer content and the effects of the inclusion of other functional excipients (EDTA, citric acid, Chlorhexidine and its salts, nalidixic acid) on the rheological properties of the Eudragit® polymers; which must be carefully controlled to optimize the operating temperatures of the co-extrusion process and the final properties of the film.

Once optimised processing conditions have been determined using knowledge gained from mDSC and thermorheological experiments, systems in which multi-layered films of varying layer thickness will be manufactured.

General background formulations are shown in the examples below.

### Example 1

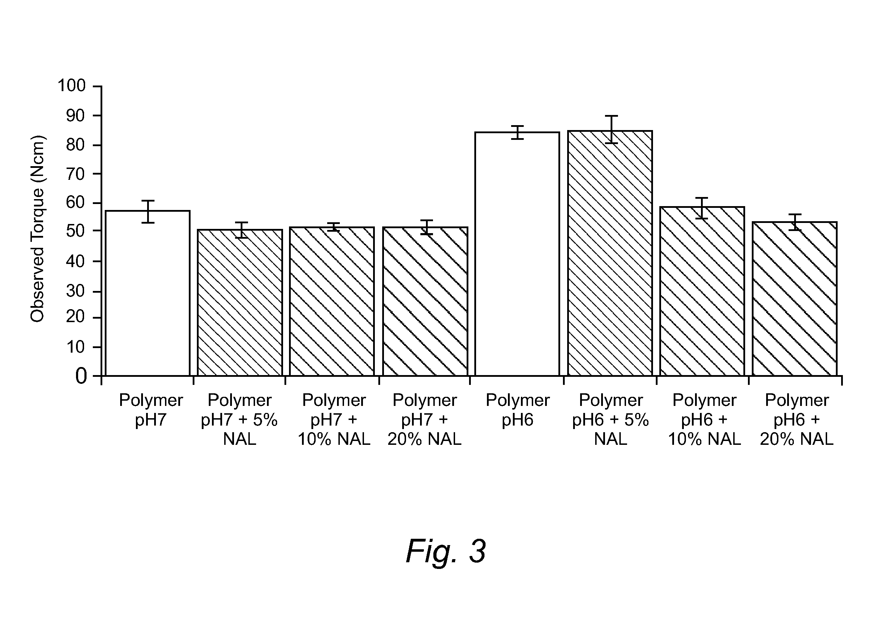

As illustrated by figure 3, the torque on the screw can be measured which provides a good indication of the viscosity and fluidity of the material within the extruder and gives an approximation of how different additives and functional agents will affect both the ease of production of the material. This also has some bearing on the final mechanical properties of the material. This graph shows a polymer that dissolves at pH 7 with a 5, 10 and 20% loading of the quinolone antibiotic Nalidixic Acid. There was little effect on the torque with increasing nalidixic acid content. However, one of the other agents, levofloxacin, showed an increase in the observed torque, showing that it made processing more difficult.

When increasing levels of nalidixic acid were added to a pH 6 dissolving polymer, there was a decrease in the torque observed on the screw, indicating that with this polymer, the drug was aiding the processing.

### Example 2

Mechanical properties of formulations can be examined using DMTA : or Dynamic Mechanical Thermal Analysis in tension mode. This involves heating the product along a temperature gradient whilst constantly oscillating it around a set point. From this data, it is possible to determine the glass transition temperature which is the temperature below which the material exists as a glassy state and above which it exists in a more flexible, rubbery state. This provides an understanding of relaxation properties of the polymer, which will have implications on the flexibility of the final product.

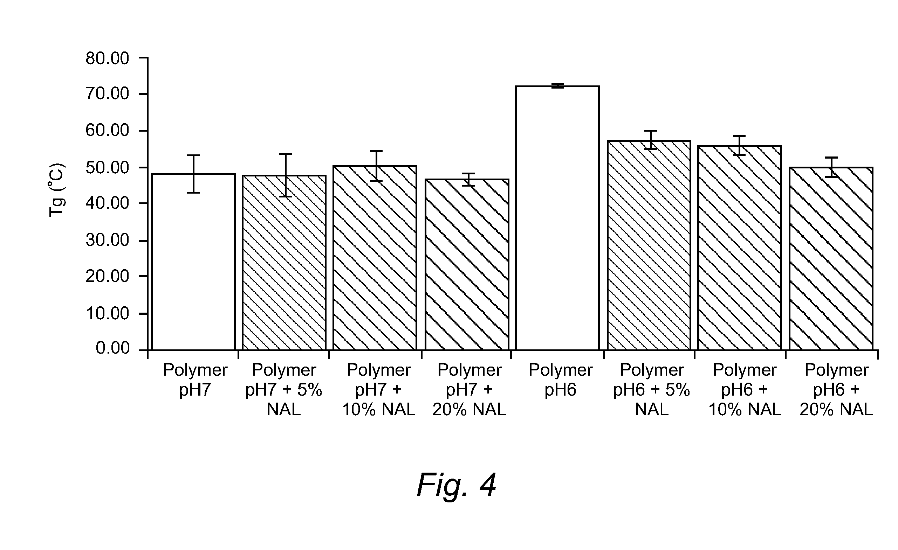

Figure 4 illustrates values which reflect those observed during processing, with Nalidixic acid causing a decrease in the glass transition temperature with the pH 6 polymer. As before, the agent which had increased the torque during processing also increased the glass transition temperature. Using strips of extruded formulations and examining them using DMTA in tension mode and complimenting the torque values, it was observed that levofloxacin increased the glass transition temperature observed, perhaps having an antiplastiicization effect.

### Example 3

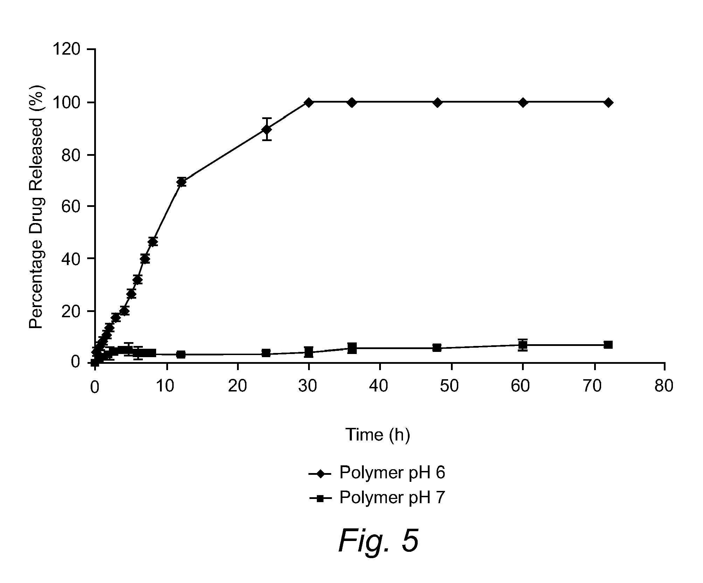

A particular formulation was tested to determine drug elution characteristics at pH 6.2. Using the formulation, a layer could be provided which constantly elutes a drug at a low level when the device is in place, but, as a failsafe device, would have the ability to switch to a more rapid response when infection is detected that is, when the pH rises. Typically, pH 6.2 is the pH of healthy, uninfected urine, whilst pH 7.8 is the pH of infected urine. As illustrated in figure 5, drug release studies of 10 % Nalidixic Acid were performed using dissolution apparatus with PBS solution at pH 6.2, to represent healthy urine, and pH 7.8 to represent infected urine.

Formulations used in this testing included a first formulation comprising Eudragit® S100 and 10% PEG 8000 and a second formulation comprising Eudragit® L100 and 20 glycerol and 20% PEG 8000.

### Example 4

Particular formulations were tested in a pH 7.8 medium, representing the infected urine. Formulations used in this testing included a first formulation comprising Eudragit® S100 and 10% PEG 8000 and a second formulation comprising Eudragit® L100 and 20 glycerol and 20% PEG 8000.

In comparison to the pH 6 dissolving polymer formulation discussed in Example 3, the pH 7 dissolving polymer releases its drug over a longer period of time.

The pH 6 dissolving polymer shows a much different release profile to the pH 7 polymer, allowing for a rapid response to the presence of infection, whilst the pH 7 dissolving polymer creates a protective barrier at the low pH values.

### Example 5

A first device comprising a pH sensitive layer including Eudragit® L100, and a second device comprising a pH sensitive layer including Eudragit® 4155F were formed. These devices allowed controlled release of the antimicrobial agents Levofloxacin and Nalidixic acid at physiological pH (approximately 6.2), and an enhanced release rate of these active agents at elevated pH levels generally associated with urinary infection (approximately 7.8).

In addition to providing a burst of antimicrobial the multi-layered films will also provide a new/clean surface that will be free from bacterial adherence.

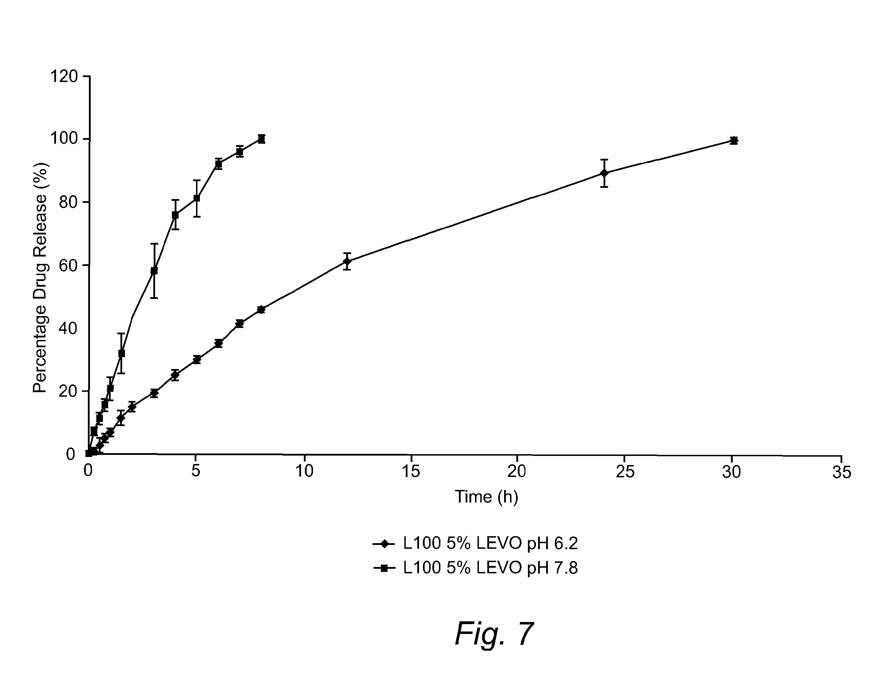

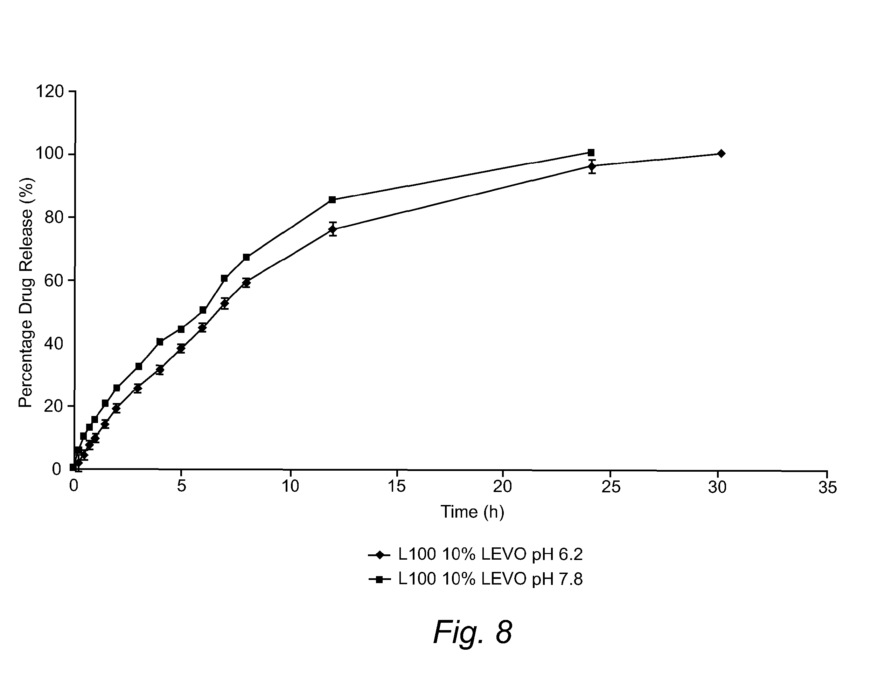

Figures 7 and 8 evidence that the release of antimicrobial from the devices can be modified by varying the pH of the release media and additionally through variation of antimicrobial loading.

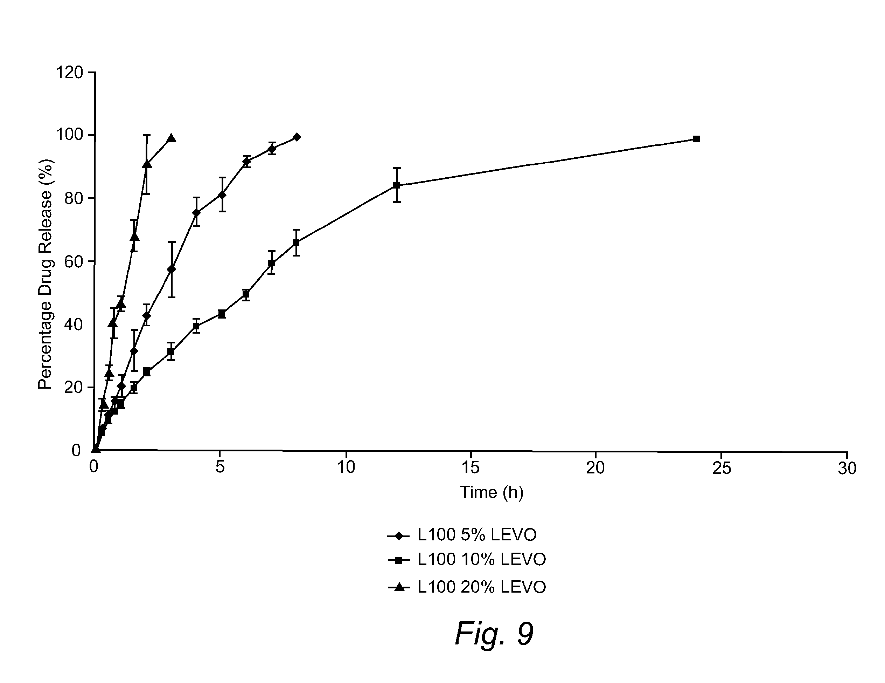

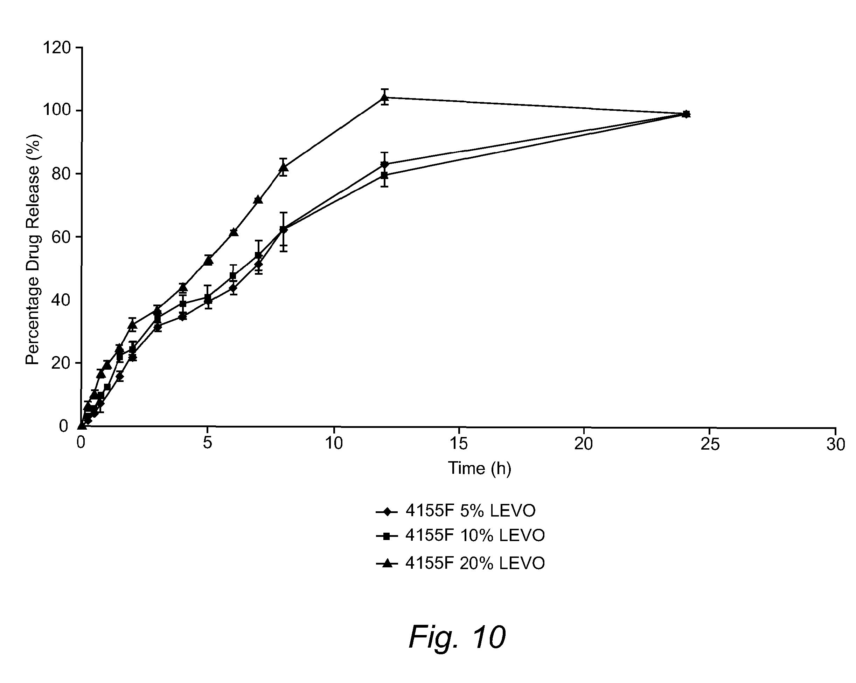

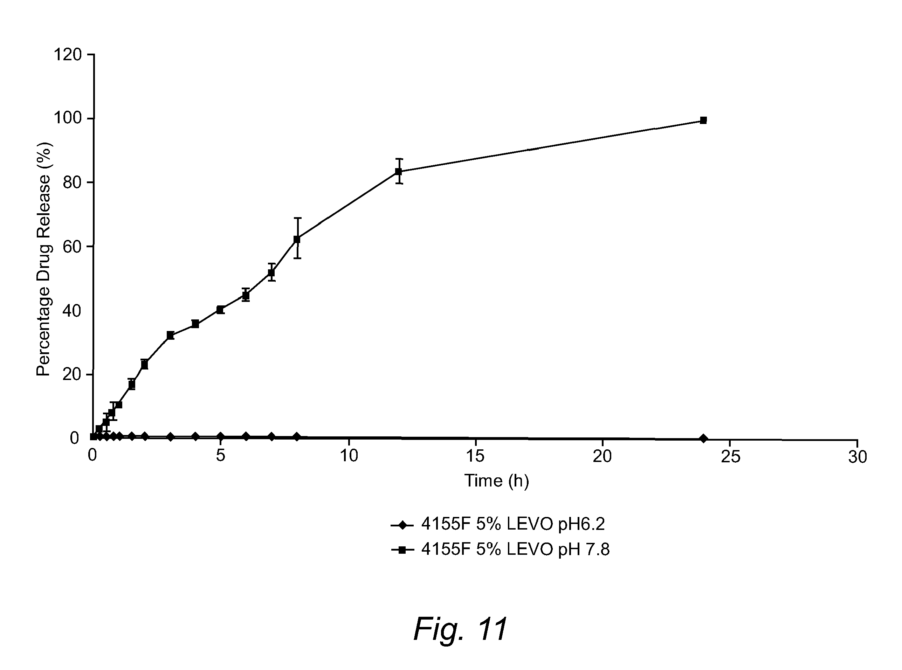

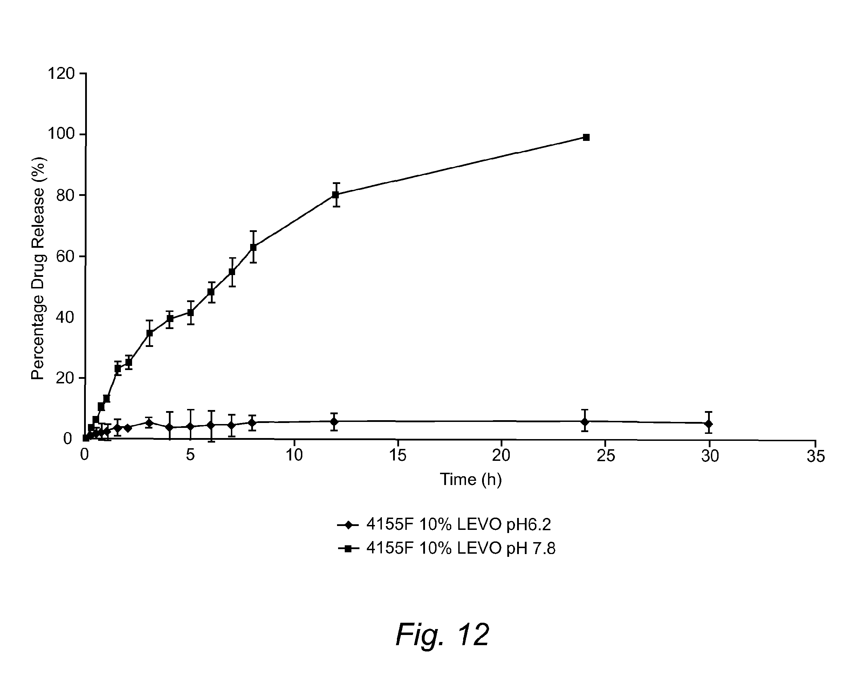

The polymeric matrix consisting of 4155F and levofloxacin has a much more controlled release of antimicrobial at pH 6.2 than L100. This is due to the fact that this polymer does not become soluble until the pH exceeds 7. This is very interesting as it will allow continuous elution of antimicrobial under 'normal' conditions. This should prevent bacterial adherence however should urease be produced (by P. *mirabilis*) and subsequently urea broken down to ammonia, the elevated pH increase will result in surface erosion and an increase in drug release rate. This is illustrated in Figures 9 to 12.

### Example 6

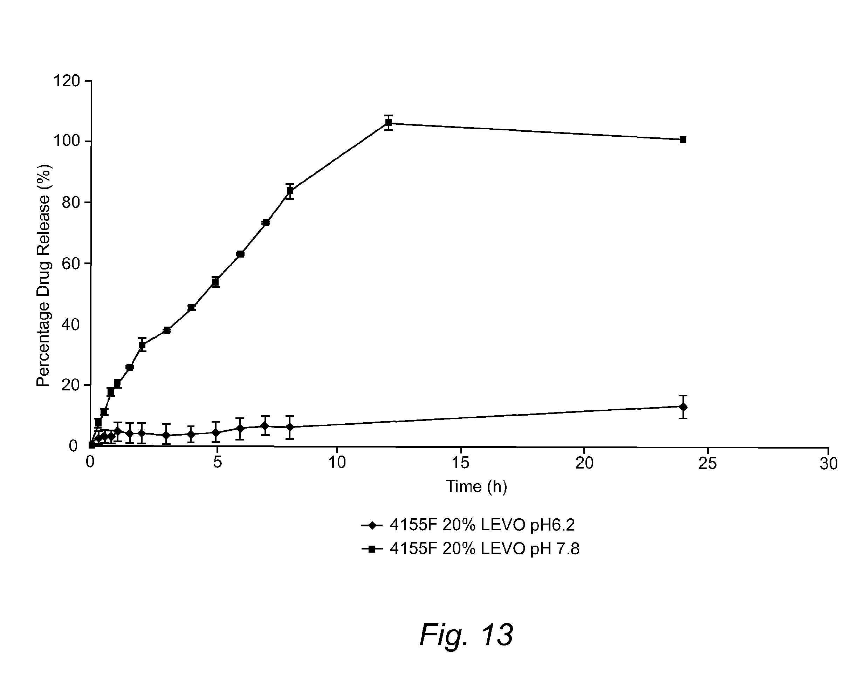

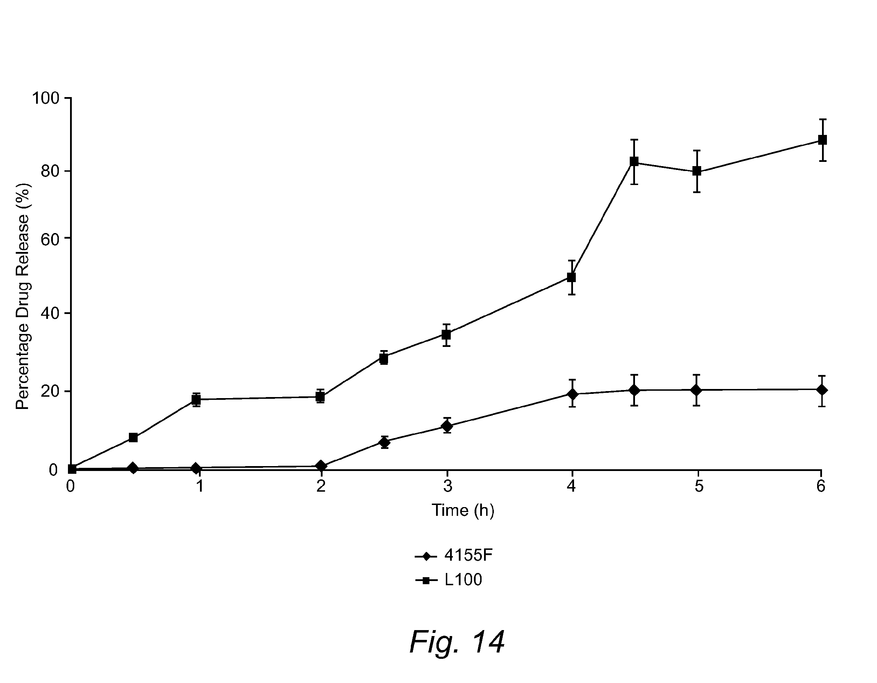

The devices of Example 5 were produced. The pH conditions surrounding the devices were maintained at pH 6.2 for 2 hours, and then adjusted to pH 7.8 for 2 hours before being adjusted back to pH 6.2 for 2 hours. Figures 13 and 14 illustrate the stop, start release profile of the devices of the present invention in response to changing pH conditions.

### Example 7

The devices of Example 5 were produced.

These studies were conducted in pH 6.2 and pH 7.8 to assess the erosion (using mass change as an indicator) of the pH sensitive layers as a function of time and also to determine the effects of antimicrobial inclusion on this process. At pH 6.2 it is clear that the device comprising Eudragit® L100 maintains mass. There is a slight increase in mass due to water uptake during the study. Eudragit® L100 which begins to erode at pH values exceeding 6 shows almost complete loss after 24 hours.

The degradation of pH sensitive layer comprising Eudragit® L100 is extremely quick at 7.8 and this was expected. The pH sensitive layer comprising Eudragit® 4155F maintains mass at pH 7.8 but additionally increases mass due to water uptake.

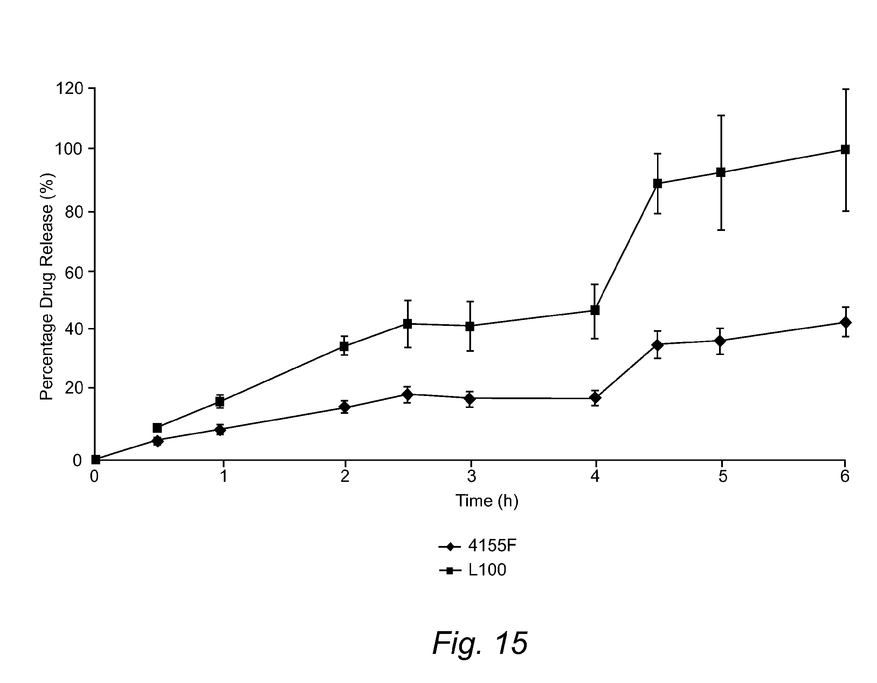

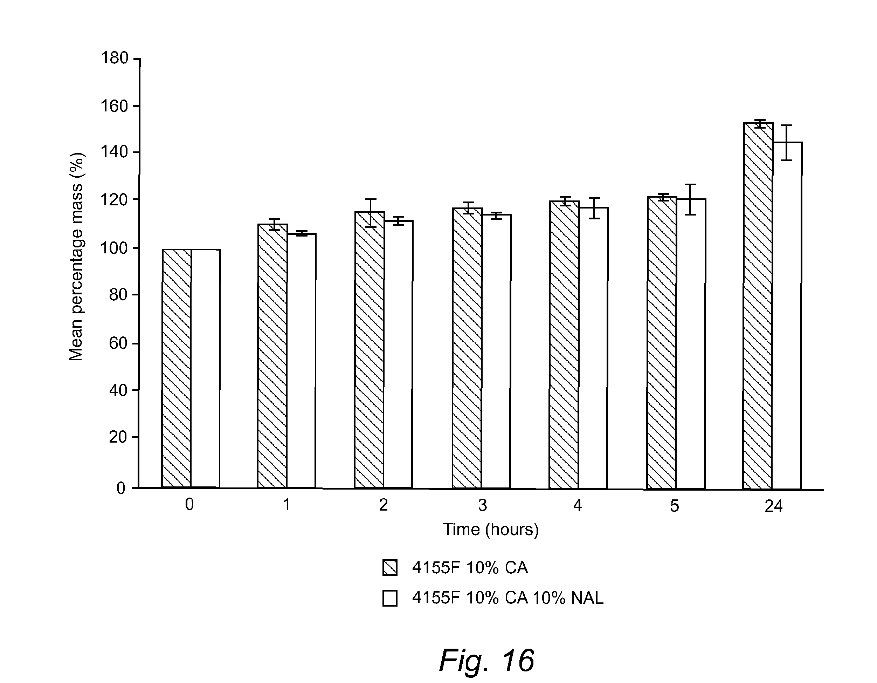

The erosion of the pH sensitive layers at pH 6.2 and 7.8 is illustrated in Figures 15 and 16.

### Example 8

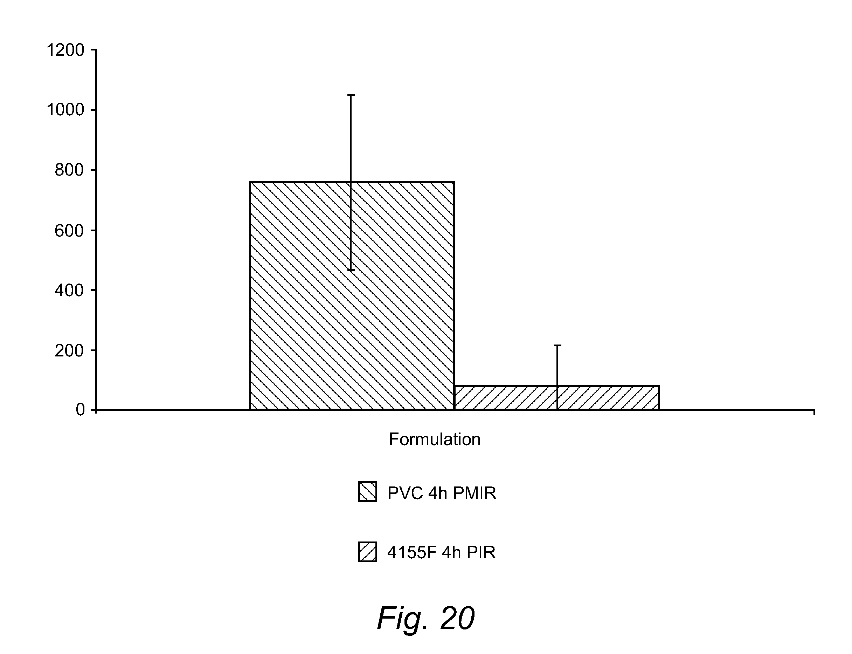

A first device was formed of PVC, and did not comprise a pH sensitive layer. A second device was formed of PVC, comprising a pH sensitive layer of Eudragit® 4155F. The two devices were immersed in artificial urine for 4 hours. The bacterial adherence of the two devices was then tested. The bacterial adherence to the first device was far greater than the bacterial adherence to the second device. The bacterial adherence was at least 8 times greater to the first device. This is illustrated in Figure 20.

## Claims

1. A device implantable or insertable into human or animal body comprising a body structure having one or more surfaces wherein at least one of the surfaces comprises at least one pH sensitive layer comprising a linear pH sensitive polymer, wherein the water solubility of the pH sensitive layer is higher at a second trigger pH than at a first physiological pH of 6.2, and wherein the linear pH sensitive polymer undergoes dissolution or erosion in an aqueous environment at the second trigger pH, **characterised in that** the pH sensitive layer incorporates buffer groups to reduce the rate of dissolution or erosion.

2. The device according to claim 1 wherein the buffers are selected from citric acid, tartaric acid, succinic acid and fumaric acid.

3. The device according to any one of the previous claims, wherein the linear polymer is selected from methacrylate polymers or cellulose polymers such as cellulose esters or cellulose ethers.

4. The device as claimed in any one of previous claims wherein the second trigger pH is either 7 or above, or 5.5 or below.

5. The device according to any one of the previous claims wherein the device comprises three layers in which two pH sensitive layers form the inner and outer layers of the device.

6. The device according to any one of the previous claims 1-13 wherein the device comprises more than three pH sensitive layers.

7. The device according to claim 6, wherein each pH sensitive layer has the same trigger pH.

8. The device according to claim 6, wherein each pH sensitive layer has a different trigger pH.

9. The device according to any one of claims 6 to 8, wherein each pH sensitive layer has the same water solubility at the second pH.

10. The device according to any one of claims 6 to 8, wherein each pH sensitive layer has a different water solubility at the second pH.

11. The device according to any one of claim 6 to 10 wherein adjacent pH sensitive layers have different rates of ionisation, or different rates of dissolution or erosion.

12. The device according to any one of claims 6 to 11 wherein different pH sensitive layers comprise different functional compounds to be released upon dissolution or erosion of the pH sensitive layers.

13. The device according to claim 12 wherein the functional compounds are either adsorbed directly to the linear polymer or disposed inside the device or otherwise associated with it via the use of one or more linker molecules or other attachment means including covalent, ionic and van der Waals bonds.

14. The device according to claims 12 or 13 wherein the functional compounds are selected from antimicrobial compounds, antibiotic compounds, levofloxacin, nalidixic acid, chlorhexidine, povidone-iodine, triclosan, urease inhibitors, EDTA and plasticizing agents.

15. The device of any preceeding claim in the form of a catheter, a stent, an implantable or insertable tube, dental braces or dentures.

16. The device of claim 16 in the form of a urinary catheter, a urinary stent, a bilary stent, a naso-gastric tube, a CAPD tube.

## Patentansprüche

1. Eine in einen menschlichen oder tierischen Körper implantierbare Vorrichtung, die eine Gehäusestruktur mit einer oder mehreren Oberflächen umfasst, wobei mindestens eine der Oberflächen mindestens eine ph-empfindliche Schicht umfasst, die ein lineares, pH-empfindliches Polymer umfasst, wobei die Wasserlöslichkeit der ph-empfindlichen Schicht bei einem zweiten Trigger-pH-Wert höher als bei einem ersten physiologischen pH-Wert von 6,2 ist und wobei das lineare ph-empfindliche Polymer in einer wässrigen Umgebung bei dem zweiten Trigger-pH-Wert einer Auflösung oder Erosion unterliegt, **dadurch gekennzeichnet, dass** die pH-empfindliche Schicht Puffergruppen einschließt, um die Auflösungs- oder Erosionsrate zu reduzieren.

2. Die Vorrichtung nach Anspruch 1, wobei die Puffer aus Zitronensäure, Weinsäure, Bernsteinsäure und Fumarsäure ausgewählt werden.

3. Die Vorrichtung nach einem beliebigen der vorstehenden Ansprüche, wobei das lineare Polymer aus Methacrylatpolymeren oder Cellulosepolymeren wie beispielsweise Ester oder Celluloseester ausgewählt wird.

4. Die Vorrichtung nach einem beliebigen der vorstehenden Ansprüche, wobei der zweite Trigger-pH-Wert entweder 7 oder darüber oder 5,5 oder darunter entspricht.

5. Die Vorrichtung nach einem beliebigen der vorstehenden Ansprüche, wobei die Vorrichtung drei Schichten umfasst, bei der zwei ph-empfindliche Schichten die innere und äußere Schicht der Vorrichtungen bilden.

6. Die Vorrichtung nach einem beliebigen der vorstehenden Ansprüche 1 - 13, wobei die Vorrichtung mehr al drei ph-empfindliche Schichten umfasst.

7. Die Vorrichtung nach Anspruch 6, wobei jede ph-empfindliche Schicht denselben Trigger-pH-Wert besitzt.

8. Die Vorrichtung nach Anspruch 6, wobei jede ph-empfindliche Schicht einen unterschiedlichen Trigger-pH-Wert besitzt.

9. Die Vorrichtung nach einem beliebigen der vorstehenden Ansprüche 1 - 6, wobei jede ph-empfindliche Schicht dieselbe Wasserlöslichkeit wie der zweite pH-Wert besitzt.

10. Die Vorrichtung nach einem beliebigen der vorstehenden Ansprüche 1 - 6, wobei jede ph-empfindliche Schicht eine andere Wasserlöslichkeit wie der zweite pH-Wert besitzt.

11. Die Vorrichtung nach einem beliebigen der vorstehenden Ansprüche 1 - 10, wobei angrenzende ph-empfindliche Schichten unterschiedliche Ionisierungsraten oder unterschiedliche Auflösungs- oder Erosionsraten besitzen.

12. Die Vorrichtung nach einem beliebigen der vorstehenden Ansprüche 1 - 11, wobei unterschiedliche pH-empfindliche Schichten unterschiedliche funktionelle Verbindungen umfassen, die bei Auflösung oder Erosion der ph-empfindlichen Schichten freigesetzt werden.

13. Die Vorrichtung nach Anspruch 12, wobei die funktionellen Verbindungen entweder direkt in das lineare Polymer aufgenommen oder innerhalb der Vorrichtung angeordnet oder sonst wie mit dieser mittels der Verwendung von einem oder mehreren Linker-Molekülen oder anderen Verbindungsmitteln verknüpft werden, darunter kovalente, ionische oder Van-der-Waals-Bindungen.

14. Die Vorrichtung nach Anspruch 12 oder 13, wobei die funktionellen Verbindungen aus antimikrobiellen Verbindungen, antibiotischen Verbindungen, Levofloxacin, Nalidixinsäure, Chlorhexidin, Povidon-Iod, Triclosan, Ureasehemmstoffen, EDTA und Plastifizierungsmitteln ausgewählt werden.

15. Die Vorrichtung nach einem beliebigen der vorstehenden Ansprüche in Form eines Katheters, eines Stents, einer implantierbaren oder einsetzbaren Sonde, einer Zahnspange oder einer Zahnprothese.

16. Die Vorrichtung nach Anspruch 16 in Form eines Blasenkatheters, eines Blasen-Stents, eines Gallen-Stents, einer naso-gastralen Sonde, einer CAPD-Sonde.

## Revendications

1. Un dispositif implantable ou insérable dans un corps humain ou animal comprenant une structure de corps qui possède une ou plusieurs surfaces où au moins l'une des surfaces comprend au moins une couche sensible au pH comprenant un polymère linéaire sensible au pH, dans lequel la solubilité dans l'eau de la couche sensible au pH est plus élevée à un second pH de déclenchement qu'à un premier pH physiologique de 6.2, et dans lequel le polymère linéaire sensible au pH subit une dissolution ou érosion dans un milieu aqueux au second pH de déclenchement, **caractérisé en ce que** la couche sensible au pH comporte des groupes tampons pour réduire le taux de dissolution ou d'érosion.

2. Le dispositif selon la revendication 1, dans lequel les tampons sont sélectionnés parmi l'acide citrique, l'acide tartrique, l'acide succinique et l'acide fumarique.

3. Le dispositif selon n'importe laquelle des revendications précédentes, dans lequel le polymère linéaire est sélectionné parmi des polymères de méthacrylate ou des polymères de cellulose tels que des esters de cellulose ou des éthers de cellulose.

4. Le dispositif tel que revendiqué dans n'importe laquelle des revendications précédentes, dans lequel le second pH de déclenchement est soit de 7 ou supérieur, soit de 5.5 ou inférieur.

5. Le dispositif selon n'importe laquelle des revendications précédentes, dans lequel le dispositif comprend trois couches dans lesquelles deux couches sensibles au pH forment les couches internes et externe du dispositif.

6. Le dispositif selon n'importe laquelle des revendications précédentes 1-13, dans lequel le dispositif comprend plus de trois couches sensibles au pH.

7. Le dispositif selon la revendication 6, dans lequel chaque couche sensible au pH possède le même pH de déclenchement.

8. Le dispositif selon la revendication 6, dans lequel chaque couche sensible au pH possède un pH de déclenchement différent.

9. Le dispositif selon n'importe laquelle des revendications 6 à 8, dans lequel chaque couche sensible au pH possède la même solubilité dans l'eau au second pH.

10. Le dispositif selon n'importe laquelle des revendications 6 à 8, dans lequel chaque couche sensible au pH possède une solubilité dans l'eau au second pH différente.

11. Le dispositif selon n'importe laquelle des revendications 6 à 10, dans lequel des couches sensibles au pH adjacentes ont des taux d'ionisation différents, ou des taux de dissolution ou d'érosion différents.

12. Le dispositif selon n'importe laquelle des revendications 6 à 11, dans lequel des couches sensibles au pH différentes comprennent des composés fonctionnels différents devant être libérés dès la dissolution ou érosion des couches sensibles au pH.

13. Le dispositif selon la revendication 12, dans lequel les composés fonctionnels sont soit adsorbés directement au polymère linéaire, soit disposés à l'intérieur du dispositif ou sinon associé à lui via l'utilisation d'une ou plusieurs molécules lieuses ou d'autres moyens de fixation comprenant des liaisons covalentes, ioniques et de Van der Waals.

14. Le dispositif selon les revendications 12 ou 13, dans lequel les composés fonctionnels sont sélectionnés parmi des composés microbiens, des composés antibiotiques, lévofloxacine, acide nalidixique, chlorhexidine, povidone-iode, triclosane, inhibiteurs d'uréase, EDTA et agents plastifiants.

15. Le dispositif de n'importe laquelle des revendications précédentes sous la forme d'un cathéter, d'un stent, d'un tube implantable ou insérable, d'arcs ou de prothèses dentaires.

16. Le dispositif de la revendication 16 sous la forme d'un cathéter ulnaire, d'un stent urinaire, d'un stent biliaire, d'une sonde nasogastrique, d'un tube pour DPCA.
